Europäisches Patentamt

European Patent Office

Office européen des brevets

(11) Publication number: **0 331 464**
**A2**

# (12) EUROPEAN PATENT APPLICATION

(21) Application number: **89302042.0**

(22) Date of filing: **01.03.89**

(51) Int. Cl.4: **C 07 K 3/08**
**C 12 P 21/02**
**// C12N9/72**

(30) Priority: **02.03.88 JP 47584/88**

(43) Date of publication of application:
**06.09.89 Bulletin 89/36**

(84) Designated Contracting States:
**BE DE FR GB IT NL SE**

(71) Applicant: **Tosoh Corporation**
**4560, Oaza Tonda**
**Shinnanyo-shi Yamaguchi-ken (JP)**

(72) Inventor: **Honma, Nobuyuki**
**35-21, Sakuradai Midori-ku**
**Yokohama-shi Kanagawa-ken (JP)**

**Hanzawa, Satoshi**
**2-7-3, Tachibanadai Midori-ku**
**Yokohama-shi Kanagawa-ken (JP)**

(74) Representative: **Kearney, Kevin David Nicholas et al**
**KILBURN & STRODE 30 John Street**
**London, WC1N 2DD (GB)**

(54) **Method of refolding urokinase precursor-like protein.**

(57) A method of refolding insoluble heterogenic protein comprising bringing urokinase precursor-like protein which is produced in the form of an insoluble heterogenic protein in host cells into contact with an aqueous alkaline solution in the presence of a protein denaturing agent, and then decreasing the pH value of the solution by adding an acid substance in the presence of a sugar.

EP 0 331 464 A2

**Description**

## METHOD OF REFOLDING UROKINASE PRECURSOR-LIKE PROTEIN

Recent progresses in gene recombination techniques have resulted in the production of heterogenic proteins from bacteria or animal cells as host. In most cases, however, the heterogenic protein thus produced is in the form of insoluble agglomerates folded into a structure of high order in which the heterogenic protein is not able to display the physiological activities of the host cells. Therefore the heterogenic protein has to be refolded into a structure in which the physiological activities are displayed.

Urokinase precursor protein is a precursor of the enzyme urokinase which has the ability to activate plasminogen, and is decomposed in blood by plasmin to turn into enzyme urokinase which has plasminogen activating ability. Thus, in recent years, there has been great interest in it as a thrombus dissolving agent.

The present invention relates to a method for refolding urokinase precursor-like protein which is produced as insoluble heterogenic protein in host cells into a form in which the physiological activities are displayed or expressed with less or no inhibition.

A method is known for refolding insoluble heterogenic protein produced in host cells, in which the insoluble heterogenic protein is denatured and solubilized in a solution of a strongly protein denaturing agent such as, for example, a 4 to 9 M solution of guanidine hydrochloride, and disulphide bonds of the protein are cleaved by sulphitolysis in the presence of a mild oxidizing agent such as sodium tetrathionate, and a sulphhydryl compound such as reduction form of glutathione is brought into contact therewith, to gradually decrease the concentration of the protein denaturing agent. However, this method eventually requires dialysis or dilution to lower the strength of the protein denaturing agent. This requirement leaves a problem in the processing when a large amount of protein is to be refolded.

Apart from the method of refolding involving the step of denaturation and solubilization of insoluble heterogenic protein by use of a protein denaturing agent and the step of lowering the strength of the protein denaturing agent, a method of refolding is known which involves the step of denaturation and solubilization by means of an aqueous alkaline solution and the step of lowering the pH of the alkaline solution. In this method, as the lowering of pH can be carried out by adding acid a large amount of insoluble heterogenic protein can be easily refolded by a much simpler operation than in the preceding known methods.

In the method of using an aqueous alkaline solution for solubilizing the heterogenic protein which is expressed as agglomerates, however, the denaturation of protein with the aqueous alkaline solution is less complete than with a protein denaturing agent and the denaturation does not extend into the inner core of the agglomerates. Therefore the degree of refolding remains relatively low. Moreover in this method, various conditions, such as selection of the alkaline compound, pH, preferable sulphhydryl compound, and the additive which acts for delicate adjustment of the properties of the solvent that influence the refolding of protein must all be determined and depend on the particular insoluble heterogenic protein which is being treated.

With these problems in mind, the present inventors conducted extensive investigations on how to refold urokinase precursor-like protein by a simple and effective operation, and finally arrived at the completion of this invention. Thus, the present invention provides a method of refolding insoluble heterogenic protein comprising bringing urokinase precursor-like protein which is produced in the form of an insoluble heterogenic protein in host cells into contact with an aqueous alkaline solution in the presence of a protein denaturing agent, and then decreasing the pH value of the said solution by adding an acid substance in the presence of sugar.

The method according to the present invention for refolding urokinase precursor-like protein is, in essence, to refold urokinase precusor-like protein, which is at least partly folded into a structure of higher order in which form it is not able to display physiological activity, into a structure in which it is at least to an increased degree able to display physiological activity. The urokinase precursor-like protein may be one of those which are partially artificially modified in addition to those produced in human kidney cells. Moreover, there is no specific limitation on the plasmid or the host employed in the operation for gene recombination.

In this invention, minute pieces of broken bacteria obtained from the host cells containing insoluble urokinase precursor-like protein by treating the cells with a homogenizer or ultrasonic waves, urokinase precursor-like protein containing material obtained from said broken bacteria by ordinary centrifugation, ultrafiltration, or gel filtration, or urokinase precursor-like protein materials emitted from bacteria are brought into contact with an aqueous alkaline solution. As the protein denaturing agent, known denaturing agents such as guanidine hydrochloride, urea, surfactants, and thiocyanates may be employed. Among them, however, urea is preferred because it has little influence on living organisms and is easy to dispose of. Concentration of the protein denaturing agent is desirably no more than that which urokinase precursor-like protein makes when it is dispersed in the solution. If a higher concentration of the denaturing agent is used, a further operation is needed to reduce the concentration of the denaturing agent. Thus, it is preferred to use a concentration of the protein denaturing agent at which urokinase precursor-like protein is not denatured, or otherwise to use a higher concentration of the denaturing agent which is reduced to a level which does not denature protein when brought into contact with an aqueous alkaline solution. For instance, since urokinase precursor-like protein undergoes denaturation at a concentration about 2 M of urea, contact of the urokinase precursor-like protein with urea preferably occurs at a concentration not greater than 2 M urea, or otherwise the contact with urea occurs at a urea concentration greater than 2 M of urea but the concentration is reduced to not greater than 2M by adding aqueous alkaline solution.

The protein denaturing agent may be dissolved beforehand in the aqueous alkaline solution, or other wise may be present together with urokinase precursor-like protein prior to the denaturation and solubilization in the aqueous alkaline solution. The latter procedure is preferred because a smaller amount of the denaturing agent is needed. Thus, in this invention, the protein denaturing agent acts to disperse the urokinase precursor-like protein, which allows the aqueous alkaline solution to enter the inner cores of the agglomerates of urokinase precursor-like protein, which, it is believed, enhances the rate of refolding.

Operations such as agitation may be conducted after the protein denaturing agent is added to the urokinase precursor-like protein.

Ordinary alkaline compounds such as sodium hydroxide and sodium carbonate may be used for the aqueous alkaline solution. However, use of an aqueous solution of weaker alkaline substances such as ammonia and organic bases such as triethylamine is preferred, since a higher rate of refolding can be expected than that which is achieved when the strongly alkaline substances mentioned above are used. Still more preferably, in order to obtain a yet higher rate of refolding, an aqueous solution of at least one organic base selected from the group consisting of monoethanolamine, diethylenetriamine, triethylenetetramine and tetraethylenepentamine may be used. The reason why an aqueous solution of organic bases leads to a higher rate of refolding of urokinase precursor-like protein is not apparent, but it is surmised that it may be because organic bases do not disturb the chaotropic properties of water. Among the organic bases, more preferred organic bases described above (that is monoethanolamine, diethylenetriamine, triethylenetetramine and tetraethylenepentamine) bring about higher rates of refolding of urokinase precursor-like protein. Although the mechanism is not yet fully understood, it is surmised that the straight chain structure of these compounds may be relevant.

An aqueous alkaline solution for denaturation and solubilization produces insufficient denaturation of urokinase precursor-like protein when the pH value is lower than 10. On the other hand, the denaturation occurs irreversibly when the pH is greater than 13, so that a pH value in the range between 10 and 13 is preferred. A pH value between 11 and 12 is most preferred for a better rate of refolding. It may be that the rate of denaturation of urokinase precursor-like protein influences the refolding in a delicate manner.

In the next stage of the process, urokinase precusor-like protein that has been solubilized is refolded. When a solution of urokinase precursor-like protein contains any pieces of bacterial body, the contaminants are preferably removed from the solution prior to the above treatment, to obtain a better result in the purification treatment that follows. Removal of the contaminants may be carried out by any ordinary method, for example, by way of centrifugation, ultrafiltration or gel filtration.

Next, the pH of the alkaline solution containin the urokinase precursor-like protein is decreased by adding an acid substance in the presence of a sugar. The sugar may be added either alone or together with the acid substance. There are no severe restrictions on the amount of sugar which may be added, but an addition of 10 to 15% by weight is preferred to avoid increases of viscosity of the mixture. Examples of sugars which may be used are glucose, fructose, and sucrose. The influence of the sugar on refolding is not clear. It may be possible that the presence of sugar somewhat affects the structure of water and renders easier hydrogen bonding between the urokinase precursor-like protein and water molecules. Whilst glucose and fructose are known as reducing sugars it is believed that the effect of sugar in this invention cannot, or cannot entirely, be due to reducing activity because a high rate of refolding can be obtained by using sucrose.

Adjustment of pH may be carried out by adding an ordinary acid substance. The acid may be selected from inorganic acids such as hydrochloric, thiocyanic, hydroiodic, and hydrobromic acids and organic acids such as acetic acid and glycine. The pH of the solution should be lower than that at the denaturation and solubilization, but preferably it needs to be adjusted finally to 8 to 9 to achieve a high rate of refolding.

Unexpected formation of disulphide bonding in the urokinase precursor-like protein can be avoided or minimized by carrying out the refolding at a higher rate. This may be achieved by adding to the solution a sulphhydryl compound such as reducing type glutathione, cysteine, 2-mercaptoethanol and dithiothreitol prior to the lowering of the pH of the solution.

If, in this invention, a high concentration of a protein denaturing agent is used and an amount of the denaturing agent which exceeds the concentration, which could denature the urokinase precursor-like protein in the solution after the refolding, is left in the solu tion, the strength of the protein denaturing agent may be reduced by dialysis or dilution as required.

This invention employs a protein denaturing agent of such a concentration as not to denature urokinase precursor-like protein and therefore the urokinase precursor-like protein produced in the form of insoluble heterogenic protein, for example from Escherichia coli as host cells, can be refolded in a simple procedure. When urea is used as the protein denaturing agent, the waste liquid after the refolding can be treated in a simpler manner than when guanidine hydrochloride is used. When the alkaline substance is selected from the group consisting of monoethanolamine, diethylenetriamine, triethylenetetramine, and tetraethylenepentamine, a higher rate of refolding can be achieved. Even higher rates of refolding can be obtained if a sulphhydryl compound is present.

Examples are presented below for the purpose of illustrating the present invention in more detail, but this invention is not restricted to these examples.

## EXAMPLE 1

Escherichia coli transformed by the plasmid which was prepared according to the method presented in Japanese Laid-Open Patent Application Sho 62-143686 were cultured in an ordinary manner, to produce insoluble urokinase precursor-like protein. The Escherichia coli containing the urokinase precursor-like protein were ground with a Gualin type homogenizer and divided into two fractions, namely insoluble and soluble fraction slurries, with a ultrafiltration membrane (TS-3000, trade name, produced by Tosoh Corporation). The fractions were employed in the following Examples.

This urokinase precursor-like protein had a molecular weight of about 46,000 and 12 disulphide bonds and the natural lysine of the 135th amino acid residue from the N-terminal had been turned into a variant of glutamine.

## EXAMPLE 2

10 ml of an 8 M urea solution was added to 10 g of the insoluble fraction slurry (corresponding to 1 g of wet bacterial body) obtained by a procedure similar to that in Example 1. The mixture was left to stand for 30 min. The pH of the solution was adjusted to about 11.5 by adding 20 ml of a 10% solution of triethylenetetramine and the solution was left to stand for a further 90 min. Then followed addition of 5 g of glucose, the mixture was left to stand for 30 min. and the pH adjusted to 9 with hydrochloric acid, to execute refolding. The reaction was carried out at room temperature.

The refolded urokinase precursor-like protein was treated with plasmin to turn it into urokinase, and then estimation was made for its activity in decomposing pyroglutamyl•glycyl•arginyl•p-nitroanilide hydrochloride which is known to be a specific substrate of urokinase. The result obtained is shown in Table 1.

## EXAMPLE 3

The procedure of Example 2 was followed except that 5 g of sucrose was used as the sugar. The result obtained is shown in Table 1.

## EXAMPLE 4

10 ml of an 8 M solution of urea was added to 10 g of the insoluble fraction slurry (corresponding to 1 g of wet bacterial body) obtained in a procedure similar to that used in Example 1, and the mixture was left to stand for 30 min. The pH of the solution was adjusted to about 11.5 by adding 20 ml of a 10% solution of triethylenetetramine and kept standing for further 30 min. Reducing glutathione was added so that its concentration be 1 mM and the mixture was left to stand for 60 min. 5 g of glucose were then added and the mixture was left to stand for 30 min. Then the pH was adjusted to 9 by adding hydrochloric acid to execute the refolding. The reaction was conducted at room temperature.

The urokinase precursor-like protein that was refolded was similarly treated as in Example 2, to convert it into urokinase the activity of which was then estimated. The result is shown in Table 1.

## EXAMPLE 5

The same procedure as was used in Example 4 was followed except that 5 g of sucrose was used as the sugar. The result obtained is shown in Table 1.

TABLE 1

| Example | Activity of urokinase (IU) |
|---------|----------------------------|
| 2 | 157000 |
| 3 | 136000 |
| 4 | 183000 |
| 5 | 164000 |

**Claims**

1. A method of refolding urokinase precursor-like protein comprising bringing urokinase precursor-like protein which is produced in the form of an insoluble heterogenic protein in host cells into contact with an aqueous alkaline solution in the presence of a protein denaturing agent, and then decreasing the pH value of the said solution by adding an acid substance in the presence of a sugar.

2. A method of refolding urokinase precursor-like protein comprising bringing urokinase precursor-like protein which is produced in the form of an insoluble heterogenic protein in host cells into contact with an aqueous alkaline solution in the presence of a protein denaturing agent, and then decreasing the pH value of the said solution by adding an acid substance in the presence of a sugar and a sulphhydryl compound.

3. A method as claimed in Claim 1 or 2, in which an aqueous solution of at least one organic base selected from the group consisting of monoethanolamine, diethylenetriamine, triethylenetetramine and tetraethylenepentamine is used as the aqueous alkaline solution.

4. A method as claimed in Claim 1, 2 or 3, in which the said sugar is glucose, fructose or sucrose or a mixture thereof.

5. A method as claimed in any one of Claims 1 to 4 in which the said protein denaturing agent is a urea solution of a concentration not greater than 2 M.